# EUROPÄISCHE PATENTSCHRIFT

(11) **EP 3 568 201 B1**
(45) Veröffentlichungstag und Bekanntmachung des Hinweises auf die Patenterteilung: **10.03.2021**
(21) Anmeldenummer: 18708325.8
(22) Anmeldetag: 11.01.2018
(51) Int. Cl.: A61N 5/10, A61B 17/00

(54) **VERFAHREN UND VORRICHTUNG ZUR RÜCKMELDUNG AN DEN PATIENTEN BZGL. SEINER ATMUNG IN DER STRAHLENTHERAPIE**
METHOD AND APPARATUS FOR PROVIDING FEEDBACK TO A PATIENT ON HIS OR HER BREATHING DURING RADIOTHERAPY
PROCÉDÉ ET DISPOSITIF PERMETTANT EN RADIOTHÉRAPIE D'ADRESSER AU PATIENT UN RETOUR D'INFORMATIONS CONCERNANT SA RESPIRATION

(30) Priorität: 12.01.2017 DE 102017200400
(43) Veröffentlichungstag der Anmeldung: 20.11.2019
(73) Patentinhaber: Opasca GmbH, 68163 Mannheim (DE)
(72) Erfinder: MACHMER, Timo, 67141 Neuhofen (DE); SWERDLOW, Alexej, 67071 Ludwigshafen (DE); LIEBSCHER, Steffen, 67063 Ludwigshafen (DE)
(74) Vertreter: Patent- und Rechtsanwälte Ullrich & Naumann
(86) Internationale Anmeldenummer: PCT/DE2018/200000
(87) Internationale Veröffentlichungsnummer: WO 2018/130253

(56) Entgegenhaltungen:
- DE-A1-102008 026 826
- US-A1- 2003 188 757
- LINTHOUT N ET AL: "Treatment delivery time optimization of respiratory gated radiation therapy by application of audio-visual feedback", RADIOTHERAPY AND ONCOLOGY, ELSEVIER, IRELAND, Bd. 91, Nr. 3, 1. Juni 2009 (2009-06-01), Seiten 330-335, XP026129653, ISSN: 0167-8140, DOI: 10.1016/J.RADONC.2009.03.019 [gefunden am 2009-05-20]

## Beschreibung

Die vorliegende Erfindung betrifft ein Verfahren und eine Vorrichtung Verfahren zur Rückmeldung an den Patienten bzgl. seiner Atmung in der Strahlentherapie, insbesondere im Atemgating, wobei dem Patienten Informationen über seinen aktuellen Atemzustand und/oder Handlungsanweisungen bzgl. seines Atems übermittelt werden.

Beim Ein- und Ausatmen verschieben sich meist die Organe im Brust- und Bauchbereich während eines Atemzyklus aufgrund der Atembewegungen. Dies beeinflusst die Genauigkeit der Bestrahlung, wodurch auch nicht zu bestrahlende, d.h. gesunde Organe bestrahlt werden.

In der Praxis wird in der Strahlentherapie atemabhängige Bestrahlung, so genanntes Atemgating oder "respiratory gating" eingesetzt. In diesem Verfahren erfolgt die Bestrahlung in einem bestimmten Atemzustand bzw. einer bestimmten Atemphase, einem so genannten Sicherheitsbereich (Gate). Meist handelt es sich hierbei um einen Bereich tiefer Atmung, in dem der Patient die Luft anhalten soll. Dieser Sicherheitsbereich ist durch bildgebende Verfahren, beispielsweise Computertomografie, bestimmbar. Durch das Atemgatingverfahren kann einerseits die Position des zu bestrahlenden Tumors/Tumorherdes stabilisiert und andererseits die Bestrahlung von gesundem Gewebe und naheliegenden Organen reduziert werden. So können beispielsweise bei Brustkrebs das Herz oder bei Lungentumoren der gesunde Lungenflügel während der Bestrahlung geschont werden.

Ein großes praktisches Problem des Atemgatingverfahrens im klinischen Einsatz besteht darin, dem Patienten eine zeitnahe und präzise Rückmeldung über seinen aktuellen Atemzustand zu vermitteln. Im Konkreten müssen dem Patienten Anweisungen gegeben werden, ob er (weiter) einatmen oder (wieder) ausatmen soll oder ob sich seine Atmung bereits im Sicherheitsbereich befindet und er den Atem zur Bestrahlung anhalten soll.

Die momentan häufigste und einfachste Variante zur Information des Patienten über seinen aktuellen Atemzustand basiert auf Sprachkommunikation durch das medizinisch-technische Personal. Das Personal gibt den Patienten Sprachkommandos in Form von "Bitte tiefer einatmen", "Atem anhalten" oder "Ausatmen". Signifikante Nachteile dieses Ansatzes sind sowohl die zeitliche Verzögerung der Sprachkommandos als auch die Unschärfe der Handlungsanweisungen aufgrund der Sprache. Insbesondere bei fremdsprachigen Patienten und/oder Personal und/oder bei starken Dialekten ist ein reibungsloser Ablauf der Behandlung häufig schwierig. Zudem stellen die Sprachkommandos einen zusätzlichen Aufwand für das Personal dar und beeinträchtigen die Konzentration und die Fokussierung auf andere, behandlungsspezifische Informationen.

Eine Alternative zu Sprachkommandos des medizin-technischen Personals stellen in der Praxis visuelle Methoden mittels aufgesetzter Virtual-Reality (VR) -Brillen oder Monitore im Behandlungsraum dar, wie in Dokumenten US 2003/188757; DE 10 2008 026826 sowie "Treatment delivery time optimization of respiratory gated radiation therapy by application of audio-visual feedback" (Linthout N. et al., Radiotherapy and Oncology, Bd. 91, Nr. 3, 1. Juni 2009, Seiten 330-335). Diese Art der Rückmeldung an den Patienten basieren auf schematischen Darstellungen seines aktuellen Atemzustands und des Sicherheitsbereichs. Der Nachteil beim Einsatz von VR-Brillen ist der zusätzliche Arbeitsaufwand für die Handhabung und die vorgeschriebene Desinfektion der Brille nach der Behandlung eines jeden Patienten. Nachteilig bei den Monitoren ist meist ein ungünstiger Blickwinkel auf den Monitor durch die Lage des Patienten auf dem Bestrahlungstisch. Sehschwächen der Patienten stellen bei beiden genannten visuellen Methoden mit schematischen Darstellungen ein weiteres Hindernis dar.

Der vorliegenden Erfindung liegt daher die Aufgabe zugrunde, eine Möglichkeit anzubieten, bei geringem Aufwand für das Personal dem Patienten zeitnahe und eindeutige Signale bzgl. seines Atemzustands zu übermitteln.

Erfindungsgemäß wird die voranstehende Aufgabe durch die Merkmale der Ansprüche 1 und 11 gelöst. Danach werden gemäß erfindungsgemäßen Verfahren zur Rückmeldung an den Patienten bzgl. seiner Atmung in der Strahlentherapie dem Patienten durch Raumsignale Informationen über seinen aktuellen Atemzustand und/oder Handlungsanweisungen bzgl. seines Atems übermittelt.

In erfindungsgemäßer Weise ist zunächst erkannt worden, dass es nicht erforderlich ist das medizinisch-technische Personal damit zu beschäftigen den Patienten Sprachkommandos zu geben und sich mit sprachlichen und/oder akustischen Hürden auseinander zu setzen. Auch wurde erkannt, dass es von besonderem Vorteil ist, den Patienten nicht etwa Informationen und Handlungsanweisungen über schematische Darstellungen an einem Monitor oder einer VR-Brille ablesen zu lassen und diese Geräte dafür handzuhaben. Ganz im Gegenteil geht die Erfindung einen völlig anderen Weg, werden ihm nämlich Informationen über seinen aktuellen Atemzustand und/oder Handlungsanweisungen bzgl. seines Atems über eindeutige, zeitnahe Raumsignale übermittelt. Durch diese Art der Rückmeldung entfallen eine zeitliche Verzögerung der Kommandos, sprachliche Hindernisse, kann sich das Personal auf andere, behandlungsspezifische Informationen konzentrieren, erkennt der Patient einfach und eindeutig die ihm übermittelten Informationen und Handlungsanweisungen bzgl. seines Atems und entfallen unnötige Arbeitsschritte, wie eine Desinfektion einer VR-Brille und/oder ein Verstellen eines Monitors. Eine ungünstige Lage des Patienten auf dem Behandlungstisch ist somit auch irrelevant.

Eine erfindungsgemäße Vorrichtung zur verbesserten Genauigkeit der Bestrahlung in der Strahlentherapie zur Anwendung eines Verfahrens nach einem der Ansprüche 1 bis 10 ist mit Anspruch 11 beansprucht. Die Vorrichtung umfasst Mittel zur Erzeugung von Raumsignalen, die dem Patienten Informationen über seinen aktuellen Atemzustand und/oder Handlungsanweisungen bzgl. seines Atems übermitteln.

Folglich ist mit dem beanspruchten Verfahren sowie mit der beanspruchten Vorrichtung eine Möglichkeit angegeben, mit der bei geringem Aufwand für das Personal dem Patienten zeitnahe und eindeutige Signale bzgl. seines Atemzustands übermittelbar sind.

Die Raumsignale können im Wesentlichen als Veränderung des Raumambientes verstanden werden. Es handelt sich bei den Raumsignalen um Raumlicht, insbesondere Raumfarbe(n), und/oder eine Veränderung der Helligkeit eines Raulichts. Zusätzlich ist Raumschall denkbar, insbesondere Tonfolge(n), und/oder eine Veränderung der Lautstärke des Raumschalls. Basierend auf dem Atemzustand bzw. der Atemphase des Patienten wird das Raumlicht und /oder der Raumschall zur Informationen über seinen aktuellen Atemzustand und/oder für Handlungsanweisungen bzgl. seines Atems eingesetzt. Hierzu kann das Raumlicht bzw. der Raumschall entsprechend des Atemzustands und/oder entsprechend Handlungsanweisungen kodiert sein. Beispielsweise kann das Erreichen des Sicherheitsbereichs durch eine Änderung der Raumbeleuchtung in grün angezeigt werden. Bei Raumschall ist es denkbar dem Patienten seine aktuelle Atemphase mittels entsprechender Modulation eines Trägersignals akustisch kodiert zu signalisieren. Eine Tonfolge sowie eine Veränderung der Lautstärke sind denkbar.

In weiter vorteilhafter Weise signalisieren die Raumsignale die Handlungsanweisungen "Einatmen" und/oder "Luftanhalten" und/oder "Ausatmen".

Eine weitere Schwierigkeit beim Atemgating besteht häufig jedoch auch darin, dass der Patient kein Gefühl dafür hat, wie weit sein aktueller Atemzustand vom erforderlichen Sicherheitsbereich entfernt ist. Dies führt regelmäßig zu Unsicherheit seitens des Patienten und erschwert die Behandlung. Um dem entgegenzuwirken, ist es hilfreich die verbleibende Dauer des Einatmens zu signalisieren, beispielsweise durch farbliche Kodierung der Differenz zum geforderten Sicherheitsbereich, so dass der Patient ein Annähern an den Sicherheitsbereich durch die Raumfarbe erkennt.

Grundsätzlich ist es wichtig, dass die erforderliche Dosis der Strahlung in das Zielgebiet, dem Tumor, eingebracht wird. Dabei wird die Strahlung mit einer festen Dosis, so genannten Monitor Units pro Zeit abgegeben. Daraus resultiert, dass für die Einbringung unterschiedlicher Dosen unterschiedlich lang bestrahlt werden muss. Dies bedeutet beim Atemgating für den Patienten, dass er je nach Dosis den Atem im Sicherheitsbereich unterschiedlich lange anhalten muss, um die Einbringung der Dosis in das Zielvolumen zu ermöglichen. In der Praxis sind heute keine Lösungen bekannt, die dem Patienten signalisieren, wie lange er den Atem anzuhalten hat. Dies führt ebenfalls häufig zu Unsicherheit und gar Nervosität der Patienten, bis hin zu Schnappatmung, was einem reibungslosen Behandlungsablauf erheblich entgegen steht. Um dies zu vermeiden und dem Patienten mehr Sicherheit zu geben und eine zügigere und präzisere Behandlung zu gewährleiten, kann zusätzlich oder alternativ die Dauer des Luftanhaltens und/oder des Ausatmens durch Raumsignale signalisiert werden. Dies lässt sich beispielsweise durch eine Kodierung der noch zu bestrahlenden Zeitspanne als Raumlicht und/oder Raumschall umsetzen.

Denkbar ist es, dass ein Sicherheitsbereich des Atems, das so genannte Gate zum Schonen nicht zu bestrahlender Organe mittels bildgebender Verfahren, insbesondere mittels Computertomografie, bestimmt wird.

In vorteilhafter Weise basieren die Raumsignale auf dem aktuellen Atemzustand. Denkbar ist eine optische Bestimmung des aktuellen Atemzustands, insbesondere durch Kamerasysteme zur Aufzeichnung der Atembewegung, und/oder eine mechanische Bestimmung, insbesondere durch Dehnmessstreifen. Dies kann vorzugsweise automatisiert erfolgen. Automatisierte Verfahren zur Bestimmung des aktuellen Atemzustands ermöglichen es, den "Hub" des Brustkorbs relativ zu einer Referenzebene während der gesamten Behandlung zu verfolgen.

Eine kontinuierliche Überwachung des Gating-Vorgangs bzw. des Atems während der Behandlung gewährleistet die Erfassung einer Vielzahl von Parametern, um individuelle, patiententypisches Atemverhalten statistisch auszuwerten. Insbesondere die individuelle Fähigkeit Luft anzuhalten ist so ermittelbar. Diese Fähigkeit ist von Patient zu Patient sehr unterschiedlich. Dies hängt von verschiedenen Faktoren ab, wie beispielsweise dem körperlichen Zustand und/oder dem Alter des Patienten.

Je nach erforderlicher Dosis der Strahlung, die bei der Behandlung eingebracht werden muss, sind unterschiedlich lange Phasen des Luftanhaltens und Bestrahlens erforderlich. Das individuelle, patiententypische Atemverhalten bzw. die Fähigkeit Luft anzuhalten führen dazu, dass die einzelnen Phasen des Luftanhaltens je nach Patient unterschiedlich lang sind und/oder unterschiedlich viele Wiederholungen des Luftanhaltens und Bestrahlens durchgeführt werden müssen. Insbesondere in Verbindung mit einem System zur Anzeige der noch ausstehenden Bestrahlungszeit fühlen sich Patienten häufig stark unter Druck gesetzt, was zu einem verändertem Atemverhalten bis hin zu Schnappatmung führen kann und die Behandlung abermals erschwert. Durch Erfassung und Auswertung des individuellen, patiententypischen Atemverhaltens kann in vorteilhafter Weise für jeden Patienten individuell die optimale Zeitspanne des Luftanhaltens und/oder die Anzahl der erforderlichen Wiederholungen des Luftanhaltens in Abhängigkeit der erforderlichen Bestrahlung ermittelt werden.

In Vorteilhafter Weise können zur Bestimmung der Zeitspanne des Luftanhaltens und/oder der Anzahl der Wiederholungen des Luftanhaltens bestehende Statistiken eines Patienten genutzt werden. Liegen jedoch über den aktuell zu behandelnden Patienten keine Statistiken bzw. Informationen über sein Atemverhalten vor, ist es denkbar, dass zur Bestimmung der Zeitspanne des Luftanhaltens und/oder der Anzahl der Wiederholungen des Luftanhaltens allgemeine, insbesondere von Alter und/oder Geschlecht abhängige Patientenstatistiken genutzt werden. So können beispielsweise aus gesammelten Statistiken für jeden Patienten mittels wissenschaftlicher Algorithmen individuell geeignete und angenehm durchführbare Phasen des Luftanhaltens und damit Zeitspannen der Bestrahlung abgeleitet werden.

Um eine besonders flexible und effiziente Behandlung zu gewährleisten, ist es denkbar, wenn das Behandlungspersonal während der Behandlung eine Feinjustierung der Zeitspanne des Luftanhaltens und/oder der Anzahl der Wiederholungen des Luftanhaltens und damit des Bestrahlens durchführen kann. Hierfür ist eine Anzeige der vorgeschlagenen Zeitspannen am Bedienplatz von Vorteil.

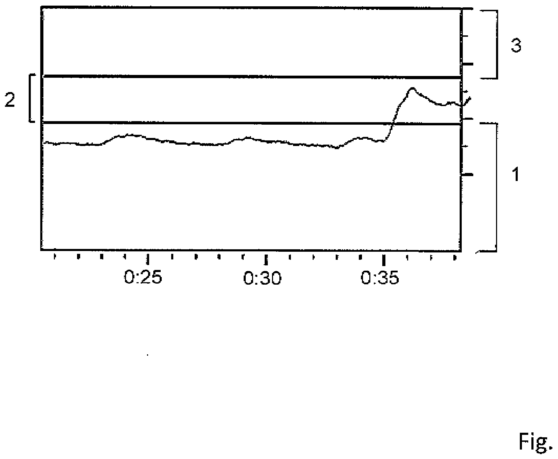

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. Die einzige Figur zeigt in einem schematischen Diagramm eine Atemkurve beim Atemgating in der Strahlentherapie, wobei dem Patienten, durch Raumsignale in Form von Raumfarben Informationen über seinen aktuellen Atemzustand und Handlungsanweisungen bzgl. seines Atems übermittelt werden. Die horizontale Achse des Diagramms zeigt den Zeitverlauf und die vertikale Achse die Tiefe der Atmung des Patienten. Der Bereich mit dem Bezugszeichen 1 ist ein Bereich zu flacher Atmung 1. In dieser Atemphase ist das Raumlicht neutral, d.h. weiß. Dies zeigt dem Patienten an, dass er noch nicht den Sicherheitsbereich - das Gate - erreicht hat und weiter einatmen muss. Erreicht er den Sicherheitsbereich, schaltet das Raumlicht auf grün und der Patient weiß, dass er die Luft anhalten soll so lange das Raumlicht die Farbe grün hat, damit die Bestrahlung erfolgen kann. Sollte der Patient zu tief eingeatmet haben, schaltet das Raumlicht auf orange, was dem Patienten signalisiert, dass seine Atmung zu tief ist und er wieder ausatmen soll.

Der Sicherheitsbereich des Atems zum Schonen nicht zu bestrahlender Organe wurde zuvor mittels Computertomografie bestimmt. Der Wechsel der Raumfarben basiert auf dem aktuellen, automatisiert erfassten Atemzustand des Patienten. Dieser wird optisch mittels eines Kamerasystems zur Aufzeichnung der Atembewegung bestimmt. Während der Behandlung wird durch kontinuierliche Überwachung der Atmung das individuelle, patiententypische Atemverhalten, insbesondere die individuelle Fähigkeit Luft anzuhalten, erfasst und statistisch ausgewertet. Hierdurch lassen sich unter Anderem die Zeitspanne des Luftanhaltens und/oder die Anzahl der erforderlichen Wiederholungen des Luftanhaltens in Abhängigkeit der erforderlichen Bestrahlung ermitteln. Eine Feinjustierung der Zeitspanne des Luftanhaltens und/oder der Anzahl der Wiederholungen während der Behandlung durch das medizin-technische Personal ist ebenfalls möglich.

Das bevorzugte Ausführungsbeispiel bringt den Vorteil mit sich, dass die Rückmeldung an den Patienten (Patientenfeedback) äußerst zeitnah erfolgt und für den Patienten einfach zu verstehen ist, unabhängig von sprachlichen Barrieren, Sehschwächen etc. Außerdem entfallen unnötige Arbeitsschritte für das Personal und kann es sich auf andere, behandlungsspezifische Tätigkeiten und Informationen konzentrieren.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken. Die Erfindung ist durch die folgenden Ansprüche definiert; andere Ausführungsbeispiele dienen lediglich den illustrativen Zwecken.

### Bezugszeichenliste

- 1: Bereich zu flacher Atmung, Farbe neutral
- 2: Sicherheitsbereich, Farbe grün
- 3: Bereich zu tiefer Atmung, Farbe orange

## Patentansprüche

1. Verfahren zur Rückmeldung an den Patienten bzgl. seiner Atmung in der Strahlentherapie, insbesondere im Atemgating, wobei sich der Patient in einem Raum befindet, wobei dem Patienten durch Raumsignale Informationen über seinen aktuellen Atemzustand und/oder Handlungsanweisungen bzgl. seines Atems übermittelt werden,
**dadurch gekennzeichnet, dass** es sich bei den Raumsignalen um Raumlicht, insbesondere Raumfarbe(n), und/oder eine Veränderung der Helligkeit eines Raumlichts handelt.

2. Verfahren nach Anspruch 1, **dadurch gekennzeichnet, dass** es sich bei den Raumsignalen zusätzlich um Raumschall, insbesondere Tonfolge(n), und/oder eine Veränderung der Lautstärke des Raumschalls handelt.

3. Verfahren nach Anspruch 1 oder 2, **dadurch gekennzeichnet, dass** die Raumsignale die Handlungsanweisungen "Einatmen" und/oder "Luftanhalten" und/oder "Ausatmen" und/oder die verbleibende Dauer des Einatmens und/oder des Luftanhaltens und/oder des Ausatmens signalisieren.

4. Verfahren nach einem der Ansprüche 1 bis 3, **dadurch gekennzeichnet, dass** ein Sicherheitsbereich des Atems zum Schonen nicht zu bestrahlender Organe mittels bildgebender Verfahren, insbesondere mittels Computertomografie, bestimmt wird.

5. Verfahren nach einem der Ansprüche 1 bis 4, **dadurch gekennzeichnet, dass** die Raumsignale auf dem aktuellen, vorzugsweise automatisiert erfassten Atemzustand basieren.

6. Verfahren nach einem der Ansprüche 1 bis 5, **dadurch gekennzeichnet, dass** der aktuelle Atemzustand optisch, insbesondere durch Kamerasysteme, und/oder mechanisch, insbesondere durch Dehnmessstreifen, bestimmt wird.

7. Verfahren nach einem der Ansprüche 1 bis 6, **dadurch gekennzeichnet, dass** durch kontinuierliche Überwachung der Atmung individuelles, patiententypisches Atemverhalten, insbesondere die individuelle Fähigkeit Luft anzuhalten, erfasst und statistisch ausgewertet wird.

8. Verfahren nach einem der Ansprüche 1 bis 7, **dadurch gekennzeichnet, dass** durch Erfassung und Auswertung des individuellen, patiententypischen Atemverhaltens die Zeitspanne des Luftanhaltens und/oder die Anzahl der erforderlichen Wiederholungen des Luftanhaltens in Abhängigkeit der erforderlichen Bestrahlung ermittelt wird/werden.

9. Verfahren nach einem der Ansprüche 1 bis 8, **dadurch gekennzeichnet, dass** zur Bestimmung der Zeitspanne des Luftanhaltens und/oder Anzahl der Wiederholungen des Luftanhaltens bestehende Statistiken und/oder allgemeine, insbesondere von Alter und/oder Geschlecht abhängige Patientenstatistiken genutzt werden.

10. Verfahren nach einem der Ansprüche 1 bis 9, **dadurch gekennzeichnet, dass** das Behandlungspersonal während der Behandlung eine Feinjustierung der Zeitspanne des Luftanhaltens und/oder der Anzahl der Wiederholungen des Luftanhaltens durchführen kann.

11. Vorrichtung zur Rückmeldung an den Patienten bzgl. seiner Atmung in der Strahlentherapie, insbesondere im Atemgating, zur Anwendung eines Verfahrens nach einem der Ansprüche 1 bis 10, mit Mitteln zur Erzeugung von Raumsignalen, die dem Patienten Informationen über seinen aktuellen Atemzustand und/oder Handlungsanweisungen bzgl. seines Atems übermitteln,
**dadurch gekennzeichnet, dass** es sich bei den Raumsignalen um Raumlicht, insbesondere Raumfarbe(n), und/oder eine Veränderung der Helligkeit eines Raumlichts handelt.

## Claims

1. Method for providing feedback to a patient with regard to his/her breathing during radiotherapy, in particular in respiratory gating, wherein the patient is located in a room, wherein by means of room signals information is transmitted to the patient relating to his/her current breathing state and/or treatment instructions with regard to his/her breathing are transmitted,
**characterised in that** the room signals are room light, in particular room colour(s), and/or a change of the brightness of a room light.

2. Method according to claim 1, **characterised in that** the room signals are additionally room noise, in particular tone sequence(s), and/or a change of the volume of the room noise.

3. Method according to claim 1 or 2, **characterised in that** the room signals signal the treatment instructions "breathe in" and/or "hold breath" and/or "breathe out" and/or the remaining duration for breathing in and/or holding the breath and/or breathing out.

4. Method according to any one of claims 1 to 3, **characterised in that** a safety range of the breathing in order to protect organs which are not intended to be irradiated is determined by means of imaging methods, in particular by means of computer tomography.

5. Method according to any one of claims 1 to 4, **characterised in that** the room signals are based on the current, preferably automatically detected breathing state.

6. Method according to any one of claims 1 to 5, **characterised in that** the current breathing state is determined optically, in particular by means of camera systems, and/or mechanically, in particular by means of strain gauges.

7. Method according to any one of claims 1 to 6, **characterised in that**, by means of continuous monitoring of the breathing, individual, patient-typical breathing behaviour, in particular the individual capacity for holding the breath, is detected and statistically evaluated.

8. Method according to any one of claims 1 to 7, **characterised in that** by detecting and evaluating the individual, patient-typical breathing behaviour, the duration of the holding of the breath and/or the number of required repetitions of the holding of the breath is/are established depending on the required irradiation.

9. Method according to any one of claims 1 to 8, **characterised in that**, in order to determine the duration of the holding of the breath and/or the number of repetitions of the holding of the breath, existing statistics and/or general patient statistics which are in particular dependent on age and/or sex are used.

10. Method according to any one of claims 1 to 9, **characterised in that** the treatment operators during the treatment can carry out a fine adjustment of the duration of the holding of the breath and/or the number of repetitions of the holding of the breath.

11. Device for providing feedback to the patient with regard to his/her breathing in radiotherapy, in particular during respiratory gating, for using a method according to any one of claims 1 to 10, having means for producing room signals which transmit information to the patient regarding his/her current breathing state and/or treatment instructions with regard to his/her breathing,
**characterised in that** the room signals are room light, in particular room colour(s), and/or a change of the brightness of a room light.

## Revendications

1. Procédé de retour au patient concernant sa respiration dans la radiothérapie, plus particulièrement dans le déclenchement de respiration, dans lequel le patient se trouve dans une pièce, dans lequel, à l'aide de signaux, des informations concernant son état actuel de respiration et/ou des instructions concernant sa respiration sont transmises au patient,
**caractérisé en ce que** les signaux sont la lumière de la pièce, plus particulièrement une ou des couleur(s) de la pièce et/ou une modification de la luminosité d'une lumière de la pièce.

2. Procédé selon la revendication 1, **caractérisé en ce que** les signaux sont en outre un son de la pièce, plus particulièrement une série de sons et/ou une modification du volume sonore de la pièce.

3. Procédé selon la revendication 1 ou 2, **caractérisé en ce que** les signaux indiquent les instructions « inspiration » et/ou « retenez votre respiration » et/ou « expiration » et/ou la durée restante de l'inspiration et/ou de la rétention de la respiration et/ou de l'expiration.

4. Procédé selon l'une des revendications 1 à 3, **caractérisé en ce qu'**une zone de sécurité de la respiration est déterminée afin de ménager les organes qui ne doivent pas être irradiés au moyen de procédés d'imagerie, plus particulièrement de tomographie assistée par ordinateur.

5. Procédé selon l'une des revendications 1 à 4, **caractérisé en ce que** les signaux sont basés sur l'état de respiration actuel, de préférence mesuré de manière automatisée.

6. Procédé selon l'une des revendications 1 à 5, **caractérisé en ce que** l'état de respiration actuel est déterminé de manière optique, plus particulièrement à l'aide de systèmes de caméras, et/ou de manière mécanique, plus particulièrement à l'aide de jauges de contrainte.

7. Procédé selon l'une des revendications 1 à 6, **caractérisé en ce qu'**une surveillance en continu de la respiration permet de mesurer et d'analyser statistiquement un comportement de respiration individuel typique d'un patient, plus particulièrement la capacité individuelle à retenir sa respiration.

8. Procédé selon l'une des revendications 1 à 7, **caractérisé en ce que** la mesure et l'analyse du comportement de respiration individuel typique d'un patient permet de déterminer le temps de rétention de la respiration et/ou le nombre de répétitions nécessaires de la rétention de la respiration en fonction de l'irradiation nécessaire.

9. Procédé selon l'une des revendications 1 à 8, **caractérisé en ce que**, pour la détermination de la durée de la rétention de la respiration et/ou du nombre de répétitions de rétention de la respiration, sont utilisées des statistiques existantes et/ou des statistiques de patients générales dépendant, plus particulièrement, de l'âge et/ou du sexe.

10. Procédé selon l'une des revendications 1 à 9, **caractérisé en ce que** le personnel soignant peut effectuer, pendant le traitement, un ajustement fin de la durée de la rétention de la respiration et/ou du nombre de répétitions de la rétention de respiration.

11. Dispositif de retour au patient concernant sa respiration dans la radiothérapie, plus particulièrement dans le déclenchement de respiration, pour l'exécution d'un procédé selon l'une des revendications 1 à 10, avec des moyens pour la production de signaux, qui transmettent au patient des informations concernant son état de respiration actuel et/ou des instructions concernant sa respiration,
**caractérisé en ce que** les signaux sont une lumière de la pièce, plus particulièrement une ou des couleur(s) de la pièce et/ou une modification de la luminosité d'une lumière de la pièce.
